## Europäisches Patentamt

## European Patent Office

## Office européen des brevets

(11) Veröffentlichungsnummer: **0 064 770**

**B1**

(12) **EUROPÄISCHE PATENTSCHRIFT**

(45) Veröffentlichungstag der Patentschrift:
**17.12.86**

(51) Int. Cl.⁴: **A 61 F 2/14**

(21) Anmeldenummer: **82104148.0**

(22) Anmeldetag: **12.05.82**

(54) Hinterkammer-Implantationslinse.

(30) Priorität: **13.05.81 DE 3119002**

(43) Veröffentlichungstag der Anmeldung:
**17.11.82 Patentblatt 82/46**

(45) Bekanntmachung des Hinweises auf die Patenterteilung:
**17.12.86 Patentblatt 86/51**

(84) Benannte Vertragsstaaten:
**AT CH DE FR GB IT LI NL SE**

(56) Entgegenhaltungen:
**DE-A-2 758 912**
**DE-B-2 607 462**
**DE-U-7 540 096**
**DE-U-7 717 661**
**GB-A-2 084 024**
**US-A-3 711 870**
**US-A-3 866 249**
**US-A-4 087 866**
**US-A-4 206 518**

(73) Patentinhaber: **INPROHOLD Establishment,**
**Bannholzstrasse 10, FL- 9490 Vaduz (LI)**

(72) Erfinder: **Schlegel, Hans- Joachim, Prof. Dr., Max-**
**Planck- Strasse 5, D-6650 Homburg (DE)**

(74) Vertreter: **Wey, Hans- Heinrich, Dipl.- Ing.,**
**Patentanwälte Müller- Börner & Wey**
**Widenmayerstrasse 49, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

## Beschreibung

Die Erfindung betrifft eine intraokulare Hinterkammer-Implantationslinse mit einem zentralen, als Sammellinse ausgebildeten Linsenkörper, dessen hintere, der Linsenkapselwand zugewandte Fläche eine stärkere Krümmung aufweist als die vordere, der Iris zugewandte Fläche und der mit einer radial nach außen ragenden Halterung versehen ist, die sich mit ihrem äußeren, auf einem Kreisbogen um den Mittelpunkt des Linsenkörpers befindlichen Rand in der Ciliarfurche des Auges abstützt. Eine solche Linse ist in der GB-A-2 084 024 beschrieben.

Vor längerer Zeit (1949) ist von Ridley in England schon versucht worden, extrakapsulär entfernte Augenlinsen durch künstliche, in die Augenhinterkammer eingebrachte Acrylglaslinsen zu ersetzen, jedoch sind diese Bemühungen aus verschiedensten Gründen, insbesondere aber aus Gründen unzureichend gestalteter Linsen und mangelhafter Werkstoffe sowie ungenügender Sterilisation mehr oder weniger gescheitert, weshalb man dann dazu überging, Implantationslinsen aus Polymethacrylsäuremethylester (PMMA), die aus einer plankonvexen Scheibe bestehen, an welcher Halterungen bzw. Stützelemente unterschiedlichster Art angebracht waren, in der Vorderkammer des Auges von Lebewesen, insbesondere von Menschen, vor der Iris anzuordnen. Aber auch diese Methode blieb der durchschlagende Erfolg versagt, weshalb in jüngerer weit Linsen der in Betracht kommenden Art mit Drahtoder Kunststoffbügeln an der Iris befestigt und gegebenenfalls noch an der Iris angenäht werden. Außer PMMA hat man auch Polyamide als Werkstoffe für derartige Linsen benutzt. Der Nachteil aller dieser Werkstoffe liegt insbesondere darin, daß die aus ihnen hergestellten Linsen nicht in chirurgisch einwandfreier Weise sterilisiert werden können. Einer zur korrekten Keimfreimachung (einschließlich Bakteriensporen) anzuwendenden Hitzesterilisation mittels überhitztem Wasserdampf oder Heißluft sind alle bisher üblicherweise verwendeten Implantationslinsen nicht gewachsen.

Demzufolge hat man sich bisher zwangsläufig damit begnügt, die Implantationslinsen unmittelbar nach ihrer Herstellung einer chemischen Flüssigkeitsentkeimung, d.h. praktisch einer Desinfektion zu unterziehen und sie dann in mehr oder minder geeigneten Flüssigkeiten ampulliert aufbewahrt. Diesen Ampullen werden die Implantationslinsen unmittelbar vor der Operation entnommen. Die Grenzen einer derartigen chemischen Flüssigkeitsentkeimung liegen im vorliegenden Falle zumindest in der Unfähigkeit, Bakteriensporen zu vernichten; außerdem reichern sich die - nicht indifferenten - Chemikalien in den Kunststoffkörpern leicht an, die sie innerhalb des Auges dann unkontrollierbar und über längere Zeiträume hinweg, einer exponentiellen Funktion folgend, verlassen. Dieses Materialverhalten ist speziell für ein Augenimplantat bedenklich.

Aus der DE-B2-2 607 462 ist eine auf den gleichen Erfinder zurückgehende Implantationslinse bekanntgeworden, die zur Befestigung an der Iris bestimmt ist und welche aus einem ganz bestimmten Silikongummi bestehen soll. Der Vorschlag der Wahl dieses Kunststoffs brachte den wesentlichen Vorteil mit sich, die Linsen nun erstmals mittels Wasserdampf oder Heißluft bei ausreichend hohen Temperaturen sterilisierbar machen zu können.

Implantationslinsen der ersten Generation waren, nachdem Ridley seine Acrylglaslinse in die Hinterkammer bzw. in den Kapselsack einsetzte, Linsen dieser vorbezeichneten Art. Infolge der dann alsbald aufgetretenen erheblichen implantieren (vgl. US-A-4 087 866), die in den unterschiedlichsten Formen ausgebildet waren. Aber auch diese Linsen, die als diejenigen der zweiten Generation bezeichnet werden, brachten nicht die Lösung der Probleme.

Linsen der dritten Generation schließlich werden diejenigen Linsen genannt, welche mittels der an ihnen angeordneten Halterungen an der Iris befestigt werden (vgl. z.B. DE-AI-2 758 912, US-A-3 866 249, US-A-4 206 518, u.a.). Die Technik zum Implantieren derartiger Linsen ist in der letzten Zeit zwar verfeinert und verbessert worden, jedoch haftet den meisten dieser Linsen nach wie vor der Nachteil an, daß sie aus PMMA oder anderen Werkstoffen bestehen, die nach anerkannten Methoden nicht sterilisierbar sind; überdies ist die Befestigung der Linsen an der Iris mit erheblichen Schwierigkeiten verbunden und muß diese Methode physiologisch nach wie vor ernsten Bedenken begegnen.

Ein weiterer Nachteil dieser bekannten Linsen wie auch der Vorderkammerlinsen (vgl. z.B. US-A-4 087 866) ist der, daß vom optischen System des Auges her betrachtet die Linse an einem nicht günstigen Ort liegt. Die aus diesem Grund empfohlenen Hinterkammerlinsen haben sich jedoch einerseits ihrer Form und andererseits ihres Werkstoffs wegen noch nicht überzeugend bewähren können.

Der Erfindung liegt die Aufgabe zugrunde, eine intraokulare Hinterkammer-Implantationslinse der eingangs genannten Art zu schaffen, die gegenüber bekannten Implantationslinsen eine wesentlich geringere mechanische Gewebsbelastung hervorruft. Das heißt, die durch die implantierte Linse unvermeidlichen Flächenpressungen auf empfindliche Gewebeteile sollen derart klein gehalten werden, daß sie physiologisch nicht mehr ins Gewicht fallen.

Die vorstehende Aufgabe für eine Implantationslinse der in Betracht kommenden Art wird erfindungsgemäß dadurch gelöst, daß die Linse in der Weise ausgebildet wir, wie dies im Kennzeichen des Patentanspruchs 1 angegeben ist.

Eine derart ausgebildete Hinterkammer-Implantationslinse ermöglicht es, die insbesondere extrakapsulär entbundene Linse an optisch günstiger Stelle funktionell voll zu ersetzen, wobei sie gleichzeitig als mechanisch stabile Trenn- bzw. Stützwand gegen den Glaskörper des Auges dient. Sie läßt sich, wie erfolgreiche Versuche gezeigt haben, in besonders vorteilhafter Weise in die Hinterkammer, d.h. zwischen dem Kapselsack und der Iris, einsetzen, wobei sie sich mit dem äußeren Rand ihrer Stützelemente in der Ciliarfurche abstützt; sie kann aber auch in der Hinterkammer in den an der Vorderseite geöffneten Linsenkapselsack eingesetzt werden.

Weitere vorteilhafte Ausgestaltungen und Merkmale der erfindungsgemäß ausgebildeten Implantationslinse gehen aus den Unteransprüchen und der nachstehenden Beschreibung einiger Ausführungsbeispiele hervor, welche anhand der Figuren 1 bis 4 der Zeichnung schematisch dargestellt sind. Es zeigen:

Fig. 1 eine Aufsicht auf eine erfindungsgemäß ausgebildete Hinterkammer-Implantationslinse in einer ersten Ausführungsform;

Fig. 2 einen querschnitt durch die Linse nach Fig. 1;

Fig. 3 eine Aufsicht auf eine Hinterkammer-Implantationslinse in einer zweiten Ausführungsform;

Fig. 4 einen Querschnitt durch eine abgewandelte Linse nach Fig. 1 bzw. Fig. 3.

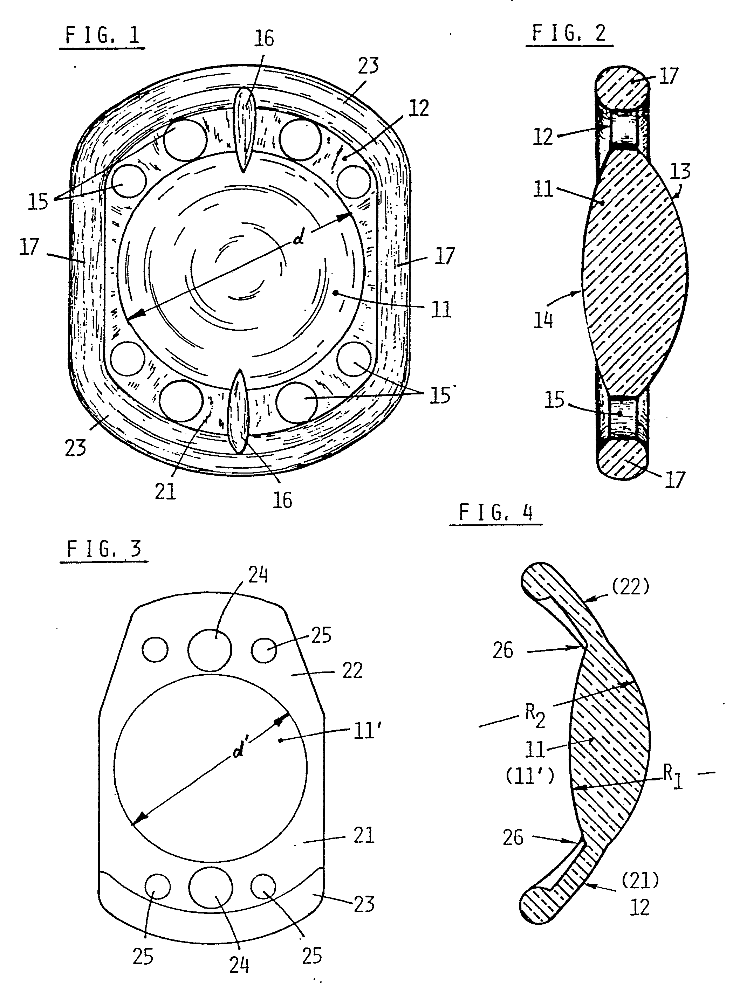

Wie aus Fig. 1 hervorgeht, ist der zentrale Linsenkörper 11, der einen Durchmesser d von vorzugsweise etwa 5,5 bis 6,0 mm aufweist, von einem sich nach außen erstreckenden Stützelement 12 umgeben, mit welchem sich die Implantationslinse an den insbesondere peripher angrenzenden Gewebeteilen im Auge abstützt. Wie insbesondere aus Fig. 4 ersichtlich, weist die hintere, der Linsenkapsel zugekehrte Fläche 13 der Linse 11 eine stärkere Krümmung auf als die vordere Fläche 14. Einerseits entspricht dies der Form der natürlichen Linse des Auges und andererseits wird durch die starke Auswölbung des Linsenkörpers 11 nach hinten die Linsenkapselhinterwand besser ausgespannt, als es der Fall wäre, wenn die Wölbung der hinteren Fläche des Linsenkörpers 11 geringer ist. Der Krümmungsradius $R_1$ der Vorderfläche 14 des Linsenkörpers 11 ist etwa 1,6 bis 2,2 mal größer als der Krümmungsradius $R_2$ der Hinterfläche 13.

In dem den Linsenkörper 11 umgebenden Stützelement 12 befinden sich Öffnungen in Form runder Löcher 15. Es kann gegebenenfalls vorteilhaft sein, am Stützelement 12 Rippen 16 anzuordnen, die dazu dienen, ein flächiges Anliegen benachbarter Gewebeflächen an der Oberfläche des Stützrings 12 zu verhindern, und zwar insbesondere in der Nähe der Löcher 15, durch die Augenkammerwasser von einer Seite der Linse auf die andere Seite hindurchtreten kann.

Am äußeren Rand des Stützelements 12 kann über den ganzen Umfang oder nur über einen Teil desselben ein Wulst 17 angeordnet sein, durch den eine beträchtliche Verringerung der auf das abstützende Gewebe einwirkenden Flächenpressung erreicht wird. Insbesondere dann, wenn man die Implantationslinse derart in die Hinterkammer einsetzt, daß sie sich mit ihrem Rand in der Ciliarfurche abstützt. Die Stärke des Wulstes 17 ist etwa 1,5 bis etwa 3,0 mal so groß wie die Stärke des Stützelements 12.

Bei der Ausführungsform nach Fig. 3, die im Prinzip den gleichen Aufbau aufweist wie diejenige nach Fig. 1 und 2, sind an dem zentralen Linsenkörper 11' zwei sich diametral gegenüberliegende Stützlappen 21, 22 angeordnet, welche dem Stützelement 12 der ersten Ausführungsform entsprechen. Die Breite dieser Stützlappen 21, 22 entspricht etwa dem Durchmesser d' des zentralen Linsenkörpers 11' oder ist etwas größer als dieser. Am Rand des unteren Stützlappens 22 kann je ein Ringwulst 23 angeordnet sein. Zwischen diesen und dem zentralen Linsenkörper 11' befinden sich Öffnungen in Form von kreisrunden Löchern 24, 25. Die größeren Löcher 24 weisen einen Durchmesser von etwa 1 bis maximal 2 mm auf, die kleineren Löcher 25 einen solchen von etwa 0,5 bis 0,7 mm. Diese Öffnungen haben einerseits die bereits erwähnte Aufgabe und andererseits erlauben sie, die Stützlappen 21, 22 bzw. die Linsen leichter zu biegen und gegebenenfalls auch umzufalten, was die Implantation erleichtert. Je nach den gewünschten Verhältnissen lassen sich mehrere größere und bzw. oder kleinere Öffnungen in den Stützlappen 21, 22 anbringen.

Vorteilhafterweise kann sich der obere Stützlappen 22 gegen seinen äußeren Rand zu etwas verjüngen; dies ist speziell dann von Vorteil, wenn man eine solche Linse in den Linsenkapselsack einsetzen will, weil sich dadurch der Rand der Kapselvorderwandöffnung, welche in diese zur Entfernung der Linsenfasern geschnitten worden ist, besser über den oberen Stützlappen 22 hinwegziehen läßt.

Wie aus Fig. 4 hervorgeht, kann das Stützelement 12 bzw. können die Stützlappen 21, 22 eine Kröpfung 26 bzw. eine mehr oder weniger starke Schräglage gegenüber der Mittenebene des zentralen Linsenkörpers 11 aufweisen, um zu erreichen, daß die hintere Fläche 13 des Linsenkörpers 11 noch weiter nach innen, d.h. gegen den Mittelpunkt des Augapfels hin zu liegen kommt, wodurch eine noch stärkere Ausspannung der Linsenkapselhinterwand erreicht wird. Zweckmäßigerweise wählt man einen unter diesem Gesichtspunkt optimalen Radius $R_2$ für die hintere Linsenfläche, den man dann für alle Implantationslinsen gleich groß läßt, und verändert hinsichtlich der gewünschten Brechkraft lediglich den Radius $R_1$ der vorderen Linsenfläche.

Wie erwähnt, stützt die in die Hinterkammer eingesetzte Implantationslinse anstelle der

natürlichen Linse den Glaskörper nach vorn ab, und zwar, was nicht zu erwarten war, auch dann, wenn die Linse intrakapsulär entfernt worden war.

Mit der erfindungsgemäßen Linse werden unter anderem folgende Vorteile erzielt: Die Linse besitzt eine große mechanische Stabilität bei niedrigem, optimalem spezifischen Gewicht des Werkstoffs, welches in etwa dem des Kammerwassers entspricht bzw. geringfügig über diesem Wert liegt. Die durch den hydrostatischen Auftrieb praktisch schwerelos gewordene Linse verursacht, soweit überhaupt eine Einwirkung auf angrenzende Gewebeteile erfolgt, eine geringstmögliche mechanische Gewebsbelastung.

Die Linse besitzt außerdem eine solch hohe Elastizität, daß das Einsetzen der Linse wesentlich erleichtert ist. Durch die Form der Linse wird auch die anatomische Linsenlage gesichert. Wo unvermeidbare Flächenpressungen auf empfindliche Gewebeteile auftreten, werden diese kleinstmöglich gehalten.

Ein noch weiterer Vorteil der erfindungsgemäßen Linse besteht darin, daß sie frei von inneren mechanischen Spannungen ist. Ihr Werkstoff ist außerdem so beschaffen, daß Verletzungen der Linsenoberfläche bei operativen Zweiteingriffen, die mitunter notwendig sind, sich nicht nachteilig auf die dioptrischen Eigenschaften der Linse auswirken. Schließlich ist die Linse physiologisch verträglich, chemisch stabil und physikalisch glasklar und läßt sich bei Temperaturen im Bereich um 200° durch Hitze sterilisieren, ohne ihre Form zu verändern.

## Patentansprüche

1. Intraokulare Hinterkammer-Implantationslinse mit einem zentralen, als Sammellinse ausgebildeten Linsenkörper (11; 11'), dessen hintere, der Linsenkapselwand zugewandte Fläche (l 3) eine stärkere Krümmung aufweist als die vordere, der Iris zugewandte fläche (14) und der mit einer radial nach außen ragenden Halterung (12) versehen ist, die sich mit ihrem äußeren, auf einem Kreisbogen um den Mittelpunkt des Linsenkörpers (11; 11') befindlichen Rand in der Ciliarfurche des Auges abstützt, dadurch gekennzeichnet, daß der mit der Halterung (12) einstückige Linsenkörper (11; 11') aus einem homogenen, glasklaren, vulkanisierten Silikonwerkstoff besteht, der ein spezifisches Gewicht zeichen 1,01 und 1,08g/cm³, vorzugsweise von etwa 1,02 g/cm³, aufweist, der eine Shore-Härte zwischen etwa 30 und 60, vorzugsweise von etwa 40 bis 50, hat und der eine Temperaturbeständigkeit ohne Formänderung der Linse und deren Teile von über 180° C während einer längeren, wenigstens 100 Stunden andauernden trockenen Wärmebehandlung besitzt, daß die Halterung von einem flachen, länglichen Stützelement (12) gebildet ist, welches aus zwei sich von dem Linsenkörper (11; 11') peripher und entgegengesetzt zueinander nach außen erstreckenden, in Implantationslage in vertikaler Richtung befindlichen Stützlappen (21, 22) besteht, deren Breite etwa dem Durchmesser (d) des Linsenkörpers (11; 11') entspricht und deren Materialstärke zwischen etwa 0,15 mm und 0,40 mm, vorzugsweise etwa 0,20 mm bis 0,23 mm, beträgt, und daß der äußere Rand des Stützelementes (12; 21, 22) auf einem Kreisbogen mit einem Durchmesser (D) zwischen ca. 9,0 mm und ca. 12,0 mm, vorzugsweise etwa 10,0 bis 11,0 mm, liegt, daß wenigstens an einem Teil des äußeren Randes des Stützelementes (12; 21, 22) sich ein vorzugsweise außen abgerundeter Wulst (17; 23) befindet, und daß sich in dem Stützelement (12; 21, 22) mehrere, über den Umfang verteilt angeordnete Öffnungen, vorzugsweise in Form runder Löcher (15; 24, 25), befinden.

2. Linse nach Anspruch 1, dadurch gekennzeichnet, daß der Wulst (17, 23) am äußeren Rand des Stützelements (12) bzw. der Stützlappen (21, 22) etwa 1,5 bis 3 mal so stark ist wie das Stützelement (12) bzw. die Stützlappen (21, 22) selbst.

3. Linse nach Anspruch 1 oder 2, dadurch gekennzeichnet, daß sich der obere Stützlappen (22) in seiner Breite nach außen hin verjüngt.

4. Linse nach Anspruch 2 oder 3, dadurch gekennzeichnet, daß das Stützelement (12) bzw. die beiden Stützlappen (21, 22) gegenüber der Linsenmittenebene eine solche Neigung aufweist bzw. aufweisen, daß der vordere Scheitel des Linsenkörpers (11; 11') hinter der Ebene liegt, in welcher sich der äußere Rand des Stützelementes (12) bzw. der Stützlappen (21, 22) befindet.

5. Linse nach Anspruch 4, dadurch gekennzeichnet, daß die Mittenebene des Linsenkörpers (11) gegenüber der Ebene, in welcher sich der äußere Rand des Stützelements (12) bzw. der Stutzlappen (21, 22) befindet, um etwa 0,5 mm bzw. 2,0 mm nach hinten versetzt ist.

6. Linse nach einem oder mehreren der Ansprüche 1 bis 5, dadurch gekennzeichnet, daß der Krümmungsradius $R_1$ der Vorderfläche (14) des Linsenkörpers (11) um etwa 1,6 bis 2,2 mal größer ist als der Krümmungsradius $R_2$ der Hinterfläche (13) des Linsenkörpers (11).

## Claims

1. Intraocular posterior chamber implant lens having a central lens body (11; 11') formed as a converging lens, whereof the rear surface (13) facing the lens capsule wall has a greater curvature than the front surface (14) facing the iris, and provided with a holding means (12) extending radially outwardly and bracing itself with its outer edge in the ciliary groove of the eye, said outer edge being disposed on a circular arc around the center point of the lens body (11; 11'), characterized in that the lens body (11; 11') integral with the holding means (12) consists of a homogeneous, crystal-clear vulcanized silicone material with a specific gravity of between 1.01 and 1.08 g/cm³, preferably of approximately 1.02 g/cm³, a Shore hardness of between 30 and 60, preferably approximately 40 to 50, and a temperature resistance of more than 180° C without deformation of the lens and its parts during a prolonged dry heat treatment lasting at least 100 hours, that the holding means is made of a flat, elongated support element (12) consisting of two support flaps (21, 22) extending peripherally from the lens body (11; 11') outwardly in opposite directions from each other and being vertically disposed when in the position of implantation, the width of said flaps approximately corresponding to the diameter (d) of the lens body (11; 11') and the material thickness thereof being between 0.15 mm and 0.40 mm, preferaby approximately 0.20 mm to 0.25 mm, and that the outer edge of the support element (12; 21, 22) lies on a circular arc with a diameter (D) of between approximately 9.0 mm and approximately 12.0 mm, preferably approximately 10.0 to 11.0 mm, that a ridge (17; 23) preferably rounded off on the outer side is located at least on one part of the outer edge of the support element (12; 21, 22) and that several openings distributed over the periphery and preferably in the shape of round holes (15; 24, 25) are provided in the support element (12; 21, 22).

2. Lens according to claim 1, characterized in that the ridge (17, 23) on the outer edge of the support element (12) or the support flaps (21, 32) is about 1.5 to 3 times as thick as the support element (12) itself or the support flaps (21, 22) themselves.

3. Lens according to claim 1 or 2, characterized in that the width of the upper support flap (22) is tapered toward the outside.

4. Lens according to claim 2 or 3, characterized in that the support element (12) or the two support flaps (21, 22) is or are inclined relative to the central plane of the lens such that the foremost point of the lens body (11; 11') lies behind the plane on which the outer edge of the support element (12) or the support flaps (21, 22) is located.

5. Lens according to claim 4, characterized in that the central plane of the lens body (11) relative to the plane on which the outer edge of the support element (12) or support flaps (21, 22) lies is displaced to the rear by approximately 0.5 mm or 2.0 mm.

6. Lens according to one or more of claims 1 to 5, characterized in that the curvature radius $R_1$ of the front surface (14) of the lens body (11) is approximately 1.6 to 2.2 times greater than the curvature radius $R_2$ of the rear surface (13) of the lens body (11).

## Revendications

1. Lentille d'implantation intraoculaire de chambre postérieure avec corps lenticulaire central (11; 11') en forme de lentille convergente dont la face postérieure (13) dirigée vers la paroi de capsule lenticulaire posséde une courbure plus forte que la face antérieure (14) dirigée vers l'iris, comportant une système de fixation (12) orienté radialement vers l'extérieur et qui s'appuie sur le sillon ciliaire de l'oeil par son bord extérieur situé sur un arc de cercle autour du centre du corps lenticulaire (11; 11,) caractérisée par le fait que le corps lenticulaire (11; 11') qui forme une seule pièce avec le système de fixation (12), est constitué d'une matière siliconée vulcanisée homogène et limpide, au poids spécifique compris entre 1,01 et 1,08 g/cm³, de préférence 1,02 g/cm³, d'une dureté-shore comprise entre 30 et 60, préférentiellement environ 40 à 50 et d'une résistance à la température telle que la lentille et ses composants supportent sans déformation un traitement thermique sec sous plus de 180°C, pendant au moins 100 heures; caractérisée aussi par le fait que le système de fixation est constitué d'un élément d'appui (21, 22) situés en direction verticale en situation d'implantation et s'étendant vers l'extérieur de façon périphérique par rapport au corps lenticulaire (11; 11'), et opposés l'un à l'autre, lobes dont la largeur correspond approximativement au diamètre (d) du corps lenticulaire et dont l'épaisseur est située entre 0,15 et 0,40 mm, préférentiellement entre environ 0,20 et 0,25 mm; caractérisée encore par le fait que le bord extérieur du système d'appui (12; 21, 22) est situé sur un arc de cercle dont le diamètre (D) oscille entre environ 9,0 mm et environ 12,0 mm, préférentiellement environ 10,0 à 11,0 mm, et que sur une partie au moins du bord extérieur du système de fixation (12; 21, 22) se trouve un collet renflé (17; 23) préférentiellement arrondi vers l'extérieur et que plusieure ouvertures sont pratiquées dans l'élément d'appui (12; 21, 22), préférentiellement sous forme de trous ronds (15; 24, 25) répartis sur la périphérie.

2. Lentille selon revendication 1, caractérisée par le fait que le collet renflé (17, 23) du bord extérieur de l'élément d'appui (12) ou des lobes d'appui (21, 22) présente une épaisseur de 1,5 à 3 fois supérieure à celle de l'élément d'appui (12) ou des lobes d'appui (21, 22) eux mêmes.

3. Lentille selon revendication 1 ou 2, caractérisée par le fait que la largeur du lobe d'appui supérieur (22) diminue vers l'extérieur.

4. Lentille selon revendications 2 ou 3, caractérisée par le fait que l'élément d'appui (12) ou les deux lobes d'appui (21, 22) présentent une inclinaison par rapport au plan médian de la lentille telle que le sommet antérieur du corps lenticulaire (11; 11') soit situé en arrière du plan dans lequel se trouve le bord extérieur de l'élément d'appui (12) ou des lobes d'appui (21, 22).

5. Lentille selon revendication 4, caractérisée par le fait que le plan médian du corps lenticulaire (11) est décalé d'environ 0,5 mm à 2,0 mm vers l'arrière par rapport au plan dans lequel se trouve le bord extérieur de l'élément d'appui (12) ou des lobes d'appui (21, 22).

6. Lentille selon une ou plusieurs des revendications 1 à 5, caractérisée par le fait que le rayon de courbure $R_1$ de la face antérieure (14) du corps lenticulaire (11) est environ 1,6 à 2,2 fois supérieur au rayon de courbure $R_2$ de la face postérieure (13) du corps lenticulaire (11).

FIG. 1

FIG. 2

FIG. 3

FIG. 4